# EUROPEAN PATENT SPECIFICATION

(11) **EP 2 133 611 B1**
(45) Date of publication and mention of the grant of the patent: **28.09.2011**
(21) Application number: 08425410.1
(22) Date of filing: 10.06.2008
(51) Int. Cl.: F16L 11/112, F16L 25/00, A61M 39/10

(54) **Hoses with connection portions**
Schläuche mit Anschlussstücken
Tuyaux avec parties de connexion

(43) Date of publication of application: 16.12.2009
(73) Proprietor: Covidien AG, 8212 Neuhausen am Rheinfall (CH)
(72) Inventor: Resca, Daniele, 41038 S. Felice Sul Panaro (IT); Zucchi, Giuseppe, 41039 San Possidonio (IT); Lodi, Marco, 41037 Mirandola (IT)
(74) Representative: Jorio, Paolo

(56) References cited:
- WO-A-2005/098302
- GB-A- 1 064 615
- US-A- 2 927 625
- US-A- 5 555 915
- US-B1- 6 305 428

## Description

The present invention relates to hoses with connection portions.

In particular, the present invention refers to respiratory circuit tubes to which the description explicitly refers without loss of generality. Respiratory circuits are used to interface in a flexible manner the tracheal or tracheostomic tube of a patient to a ventilation system.

Currently, the types of tube used for said purpose are spiral tubes, generally made of PVC and consisting of a flexible flat part and a rigid reinforcement rib wound in a continuous spiral; or corrugated tubes, generally made of continuously blown polyethylene or polypropylene and provided with inserts to facilitate the connection to dedicated fittings; or extensible tubes, also generally made of continuously blown polypropylene and provided with inserts to facilitate connection to dedicated fittings. The latter are supplied packed. Such hoses are known from documents GB 1,064,615, US 5,555,915 or US 2,927,625.

Condensation may form in respiratory circuit tubes and this represents a vehicle for the bacteria present inside the patient and the circuit itself, in addition to constituting a serious danger for the patient as it can get into the lungs, preventing correct functioning thereof. In said regard the preferred type of tubes are spiral tubes, as they have transparent walls and, since the walls are smooth, there are no dead areas that can favour the accumulation of condensation, a phenomenon which occurs, on the other hand, in corrugated tubes.

Generally, spiral tubes have flexible terminal connections which are applied to fittings by means of solvent gluing.

The object of the present invention is to produce spiral tubes comprising terminal connection portions that do not require gluing for application to the fittings.

The subject of the present invention is a hose comprising a tubular wall, a rib wound spirally on said tubular wall and a core of rigid material; said hose also comprising one or more duct portions and one or more connection portions; in said connection portions said rib being wound spirally with a narrower pitch than in said duct portions, according to the preamble of claim 1.

According to the present invention, the core made of rigid material increases its diameter as it moves away from a connection portion towards inside of a duct portion.

The following examples serve for illustrative non-limiting purposes, for a better understanding of the invention with the help of the figures of the accompanying drawing, in which:
figure la illustrates a tube according to the prior art as produced by the extruder;
figure 1b illustrates a tube obtained from the tube illustrated in figure 1a;
figure 1c is a longitudinal section of the tube of figure 1b; figure 2 is a longitudinal section of a tube according to the present invention; and
figures 3a-3d are respective four sections of the rib of the tube of figure 2.

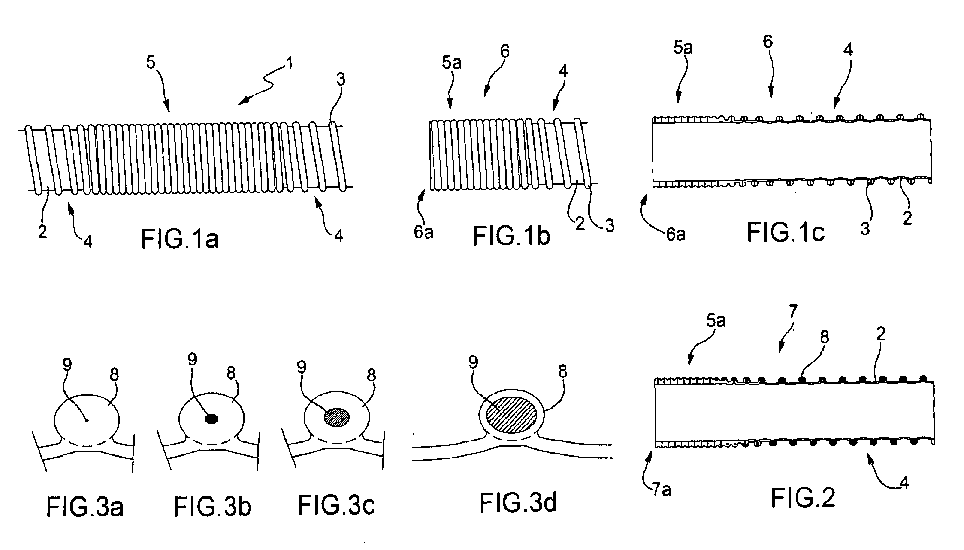

In figure 1a, the number 1 indicates overall a hose which is not subject of the present invention.

The tube 1 is produced by means of extrusion and comprises a smooth tubular wall 2 and a rib 3 made of the same material as the tubular wall 2 around which the spiral is wound.

The tube 1, as obtained by the extruder, comprises a succession of duct portions 4 and connection portions 5, only one of which can be seen in figure la. Each of the duct portions 4 has a constant pitch different from zero and common to the traditional spiral tubes, while each of the connection portions 5 comprises a rib 3 wound spirally with a pitch reduced to a minimum, almost to zero.

In figure 1b and 1c, 6 indicates a tube obtained from the tube 1 following a cutting operation in the connection portions. In this way, the tube 6 produced comprises a connection portion 5a deriving from a part of the connection portion 5 of the tube 1, and arranged at one end 6a of the tube 6 so that it can connect directly to the connector elements of a respiratory circuit.

As is evident from figure 1c, the pitch reduced to a minimum almost to zero of the rib 3 of the connection portions 5 and 5a results in a thickening of the wall of the tube.

In figure 2 an embodiment of the tube according to the present invention is indicated overall by 7.

The parts of the tube 7 equal to those of the tube 1 will be indicated by the same numbering and will not be described again.

The tube 7 is distinguished from tube 1 in relation to a different structure of the rib which in this case is indicated by 8. The rib 8 present in the duct portions 4 comprises a rigid core 9. According to the present invention, as shown in figure 2 and as illustrated in detail in the figures 3a-3d, the thickness of the rigid core 9 increases as it moves away from the connection portion 5a located at one end 7a of the tube 7. In other words, in the duct portions 4 the rib 8 near the connection portions 5a has a rigid core 9 with very small diameter (figure 3a) which increases (figures 3b-3d) as it moves towards the inside of the duct portion 4 where, once a certain dimension has been reached, it remains constant.

The tubes according to the invention can be produced by extrusion using three different extruders. A first extruder is responsible of the extrusion of tubular wall 3, while a second and a third extruder are respectively responsible of the extrusion of rigid core of the rim and of outer part of the rim. In particular, by changing the speed of a extruder screw of the second and the third extruders, it is possible to dose the quantity of both the rigid core material and the outer material of the rim, while maintaining unchanged the rib external diameter. In other words, the combination of the coextrusion variability together with a variable pitch mandrel, allows a progressive decrease of pitch during extrusion without stops coupled with decrease of rigid core screw velocity and increase of soft material screw velocity. The tubes of the present invention offer the advantage to guarantee in one whole piece the same properties as the hoses of the prior art about rigidity and flexibility of the different portions of the tube itself.

In particular, the tubes of the present invention do not require the use of specific fittings for their assembly in the respiratory circuit and, therefore, do not require gluing operations. In fact, the tubes subject of the present invention permit assembly of the circuits by means of a simple insertion operation. Furthermore, the tubes subject of the present invention can be made of materials different from PVC. Lastly, once the tube comprising a plurality of connection portions has been produced by extrusion, tubes can be produced with the terminal connection portion of the required length according to where the cut is made.

## Claims

1. Hose (1) comprising a tubular wall (2) and a rib (8) wound spirally on said tubular wall (2), the rib comprising a core (9) of rigid material; said hose (1) comprising one or more duct portions (4) and one or more connection portions (5); in said connection portions (5) said rib (8) being wound spirally with a narrower pitch than on said duct portions (4); said hose being **characterised in that** said core (9) of rigid material increases its diameter as it moves away from a connection portion (5) towards inside of a duct portion (4).

2. Hose (1) as claimed in claim 1, **characterised in that** the diameter of said core (9) of rigid material remaining constant once a certain dimension has been reached.

3. Hose (6; 7) comprising at least one connection portion (5a) arranged at one end (6a; 7a) and at least one duct portion (4); said hose (6; 7) being **characterised in that** it is produced by means of a cutting operation in one connection portion (5) of the hose (1) as claimed in claim 1 or 2.

4. Method for producing by extrusion a hose (1) as claimed in claim 1 or 2; said method being **characterised by** using three different extruders, which are responsible respectively of the extrusion of the tubular wall (3), of the core (9) of rigid material and of an outer material of the rim.

## Patentansprüche

1. Schlauch (1) mit einer röhrenförmigen Wandung (2) und einer Rippe (8), welche spiralförmig auf der rohrförmigen Wandung (2) gewunden ist, wobei die Rippe einen Kern (9) aus einem festen Material aufweist; wobei der Schlauch (1) einen oder mehrere Leitungsabschnitte (4) und einen oder mehrere Verbindungsabschnitte (5) aufweist; wobei die Rippe (8) in den Verbindungsabschnitten (5) spiralförmig mit einem geringeren Abstand als auf den Verbindungsabschnitten (4) gewunden ist; wobei der Schlauch **dadurch gekennzeichnet ist, dass** der aus einem festen Material bestehende Kern (9) einen Durchmesser aufweist, welcher sich von einem Verbindungsabschnitt (5) weggerichtet in Richtung des Inneren eines Verbindungsabschnitts (4) hin vergrößert.

2. Schlauch (1) nach Anspruch 1, **dadurch gekennzeichnet, dass** der Durchmesser des aus einem festen Material bestehenden Kerns (9) konstant bleibt, sobald eine vorbestimmte Abmessung erreicht worden ist.

3. Schlauch (6, 7), welcher wenigstens einen Verbindungsabschnitt (5a) aufweist, der an einem Ende (6a; 7a) und an wenigstens einem Verbindungsabschnitt (4) angebracht ist; wobei der Schlauch (6; 7) **dadurch gekennzeichnet ist, dass** er mittels eines Schneidvorgangs in einem Verbindungsabschnitt (5) des Schlauchs (1) wie in dem Anspruch 1 oder 2 beansprucht hergestellt ist.

4. Verfahren zum Herstellen eines Schlauchs (1) nach Anspruch 1 oder 2 mittels Extrusion; wobei das Verfahren **gekennzeichnet ist durch** den Schritt des Verwendens von drei verschiedenen Extrudern, welche jeweils für die Extrusion der rohrförmigen Wandung (3), des aus einem festen Material bestehenden Kerns (9) und eines Außenmaterials der Berandung vorgesehen sind.

## Revendications

1. Tuyau (1) comprenant une paroi tubulaire (2) et une nervure de renforcement (8) enroulée en spirale sur ladite paroi tubulaire (2), la nervure de renforcement comprenant une âme (9) en matériau rigide ; ledit tuyau (1) comprenant une ou plusieurs portions de canalisation (4) et une ou plusieurs portions de connexion (5) ; ladite nervure de renforcement (8) étant enroulée en spirale dans lesdites portions de connexion (5) avec un pas plus étroit que sur lesdites portions de canalisation (4) ; ledit tuyau étant **caractérisé en ce que** ladite âme (9) de matériau rigide augmente de diamètre à mesure qu'elle s'éloigne d'une portion de connexion (5) vers l'intérieur d'une portion de canalisation (4).

2. Tuyau (1) selon la revendication 1, **caractérisé en ce que** le diamètre de ladite âme (9) de matériau rigide reste constant une fois qu'une certaine dimension a été atteinte.

3. Tuyau (6 ; 7) comprenant au moins une portion de connexion (5a) disposée à une extrémité (6a ; 7a) et au moins une portion de canalisation (4) ; ledit tuyau (6 ; 7) étant **caractérisé en ce qu'**il est produit au moyen d'une opération de coupe dans une portion de connexion (5) du tuyau (1) selon la revendication 1 ou 2.

4. Procédé de production par extrusion d'un tuyau (1) selon la revendication 1 ou 2 ; ledit procédé étant **caractérisé par** l'utilisation de trois extrudeuses différentes, qui sont respectivement responsables de l'extrusion de la paroi tubulaire (3), de l'âme (9) de matériau rigide et d'un matériau extérieur de la bordure.
